# EUROPEAN PATENT APPLICATION

(11) **EP 3 485 852 A1**
(43) Date of publication of application: **22.05.2019**
(21) Application number: 16908854.9
(22) Date of filing: 14.07.2016
(51) Int. Cl.: A61F 5/01, A41D 13/05, A61F 5/02

(54) **UPPER BODY SUPPORT APPARATUS AND UPPER BODY SUPPORT METHOD**

(71) Applicant: Helinx Japan Corp., Tokyo 107-0052 (JP)
(72) Inventor: NISHI, Taketane, Chigasaki-shi Kanagawa 253-0053 (JP)
(74) Representative: Brookes IP
(86) International application number: PCT/JP2016/070881
(87) International publication number: WO 2018/011952

(57) **Abstract**

To provide a practical technique for assisting the motion of the upper body of a subject or improving a posture.

An upper body assisting device includes shorts 19 and an upper body strap 30. The upper body strap is secured to the shorts 19 at the front of the left thigh, and passes from the left thigh along a location near the left greater trochanter to the left ilium, the right shoulder, the front of the right underarm, the back of the left underarm, the left shoulder, and the right ilium. The upper body strap passes from the right ilium to the front of the right leg along the greater trochanter, and then is secured to the shorts 19 at the front of the right leg.

## Description

### Technical field

The present invention relates to techniques for assisting the motion of the upper body of a subject or improving a posture.

### Background art

Human beings are capable of upright bipedal walking and human bodies have evolved adaptations to do this. However, bipedal walking on the earth, where gravity exists, is inherently unnatural, as evident from the fact that many other mammals exhibit quadrupedal walking. Accordingly, the human bodies continually ensure a heavy burden, even in daily life.

For example, the human upper body should inherently be vertically upright on the pelvis. However, in many actual cases, the human upper body is slightly inclined forward. Such a posture commonly called "stoop" causes various harmful effects as is well known.

The following harmful effects caused by the stoop are well known. For example, metabolism deterioration, shoulder stiffness, neck stiffness, headache, and disorders in a circulatory system, a respiratory system, a gastrointestinal system, and a gynecological system are liable to occur. Moreover, mental instability caused by disorders in an autonomic system may occur.

Moreover, not only the distortion in the forward and backward directions described above is given. For example, at the time of walking, the human body swings in the rightward and leftward directions, and rotates in plan view. Such swing in the rightward and leftward directions or rotation may also give a burden on the human body.

### Summary

### Problems to be solved

There have been proposed various devices for assisting the motion of the upper body or improving a posture. However, most of those devices focus only on elimination of the stoop.

Thus, in actual circumstances, there exists no effective device for assisting the motion of the upper body or improving a posture.

An object of the present invention is to provide a practical technique for assisting the motion of the upper body of a subject or improving a posture.

### Means to solve the problems

In order to solve the aforementioned problems, the present inventor proposes the following inventions.

The invention is an upper body assisting device including: an upper body strap being a strip-shaped substance having at least a length that allows the upper body strap to connect a first point, which is a predetermined point with respect to a predetermined point on a line that extends from the right greater trochanter of a subject in the forward and backward directions by 10 cm, and a second point, which is a predetermined point with respect to a predetermined point on a line that extends from the left greater trochanter of the subject in the forward and backward directions by 10 cm, with each other along a path: from the first point to the left shoulder of the subject, going along the back side of the body of the subject; from the left shoulder to the left underarm of the subject, going along the front side of the body of the subject; from the left underarm to the right underarm of the subject, going along the back side of the body of the subject; from the right underarm to the right shoulder of the subject, going along the front side of the body of the subject; and from the right shoulder to the second point, going along the back side of the body of the subject, or vice versa; and securing means configured to removably secure, under a state in which the upper body strap is tensioned, both ends of the upper body strap to securing points, which are located at the same positions as those of the first point and the second point or at predetermined positions where are lower than the first point and the second point and along the body of the subject.

Such upper body assisting device put on a subject helps retract the shoulders of the subject backward and thereby allow him/her to assume a good posture with the chest puffed out. Against the swing and rotation of the body of the subject while walking, the upper body assisting device exerts a force to return the swinging or rotating body to its original position, providing effects in reducing the swing and rotation while assisting walking.

The subject can stay for a long period of time (for example, 20 minutes or longer, or 2-3 hours or more in daily life) with the upper body assisting device worn.

As used herein, the term "connecting" the upper body strap to the first point and the second point includes a case in which a part of the upper body strap is at least brought into contact with the first point or the second point.

The upper body strap may be stretchable in a lengthwise direction thereof. With this, the effect of the upper body assisting device described above is more emphasized.

As used herein, the term "stretchable" indicates that a rate of elongation {[(length after elongation - original length)/original length] × 100(%)} is ≥10% when measured by securing the strip-shaped substance across its entire width at predetermined positions, with 30-cm intervals and exerting a load of 2 kg downward at the center between the secured positions of the strip-shaped substance. The aforementioned method of measuring the rate of elongation is referred to as Measurement Method A in this application. Similarly, as used herein, the term "less easily stretchable" with regard to the strip-shaped substance indicates that the rate of elongation of the strip-shaped substance is ≤5% when measured using the Measurement Method A.

It is preferred that the stretchability of the upper body strap in this application be 30-50%, more preferably 40%±5%. When the rate of elongation is set within those ranges, the excessive burden on the subject is prevented while the effect of the upper body assisting device is ensured.

Under the state in which the upper body strap is tensioned as described above, both ends of the upper body strap are removably secured by securing means at securing points which are located at the same positions as those of the first point and the second point or at predetermined positions where are lower than the first point and the second point and along the body of the subject. The securing points at which the upper body strap is secured by the securing means may be the same as the first point and the second point described above, or may be lower than the first point and the second point. In other words, a path of the upper body strap at a portion thereabove is determined, but as long as the path passes through both the first point and the second point, one of the first point and the second point may be set as a starting point and another of the first point and the second point may be an ending point, or the both ends may be secured at any appropriate position below passing through the first point and the second point.

The securing means may be provided integrally with or separately from the upper body strap. When the securing means is provided separately from the upper body strap, the upper body strap is configured so as to be removably secured with respect to the securing means.

The securing means may be cloth to be secured to the body of the subject, and the securing points may be provided on the cloth. The upper body strap is a strip-shaped substance as described above. Therefore, the upper body strap can be tightly fitted to the body of the subject. When the securing means is formed of cloth, the securing means can also be tightly fitted to the body of the subject. Therefore, when the upper body assisting device is put on the subject, the upper body assisting device is less liable to be felt unpleasant by the subject, or is less liable to hinder the motion of the subject. In this case, the securing means may be configured to have, for example, a half-pant style.

The securing means may be a strip-shaped substance, and the securing points may be provided on the strip-shaped substance being secured to the body of the subject in a state of being wrapped around the body of the subject. Such securing means can also be tightly fitted to the body of the subject. Thus, an effect similar to that given in the case described above may be attained.

For example, the securing means being the strip-shaped substance may include: a right leg strap to be secured in a state of being wrapped around the right thigh; and a left leg strap to be secured in a state of being wrapped around the left thigh. In this case, the securing points may be provided on the right leg strap and the left leg strap, respectively.

In this case, further effects can be expected for the right leg strap and the left leg strap.

Approximately 70% of the thigh muscles are lateral rotators that externally rotate the femur and approximately 30% are medial rotators that internally rotate the femur. Since the lateral rotators constitute most of the thigh muscle complex, the thigh muscles tend toward external rotation in daily life mainly due to the structure of the muscles. As used herein, the lateral rotation of the thigh muscles about the femur (specifically, "lateral rotation" means that the muscles of the front of the thigh move outward) is referred to as "external rotation" of the thigh muscles. Likewise, as used herein, the medial rotation of the thigh muscles about the femur (specifically, the "medial rotation" means that the muscles of the front of the thigh move inward) is referred to as "internal rotation" of the thigh muscles. Although external rotation of the thigh muscles is not always dangerous, excessive external rotation may induce health problems such as pelvic opening causing lower back pain, bowed legs, difficulties in sit-to-stand movement because of the loss of thigh strength during the sit-to-stand movement. In particular, during walking, the internal rotators should play a major role, and walking can be difficult when the external rotators become dominant. Such problems sometimes occur at a young but become more remarkable with increasing age. Such problems have been recognized, but no realistic solution by which the problems can be solved is known.

The right leg strap being a strip-shaped substance exerts, on the right thigh muscles, a force making the right thigh muscles rotate internally by wrapping the right leg strap around the right thigh at a predetermined compression pressure in use. The left leg strap being a strip-shaped substance exerts, on the left thigh of the subject, a force making the left thigh muscles rotate internally by wrapping the left leg strap around the left thigh at a predetermined compression pressure in use. Consequently, the internal rotation of the right thigh muscles and the left thigh muscles improves. For example, the right leg strap makes the thigh muscles internally rotate by being wrapped around the right thigh in a direction of internal rotation of the thigh muscles and secured thereat, or by being shifted in a direction of internal rotation of the thigh muscles after the right leg strap is secured around the right thigh with a predetermined compression pressure. The same principle applies to the left leg strap. The right and left leg straps each of which is the strip-shaped substance can be worn on the legs for a relatively long period of time (e.g., ≥20 minutes, or for 2-3 hours in daily life). Consequently, the internal rotation of the thigh muscles of the subject improves better than when the subject performs exercise or stretching or when the subject has received massage. Moreover, the effect of such internal rotation can be sustained for a longer period of time.

The securing means being the strip-shaped substance may include: a right leg strap to be secured in a state of being wrapped around the right thigh; a left leg strap to be secured in a state of being wrapped around the left thigh; and a waist/hip strap being a strip-shaped substance having a length that allows the waist/hip strap to connect the back of the right leg strap worn on the right leg of the subject and the back of the left leg strap worn on the left leg of the subject with each other along a path: from the back of the right leg strap to the outer side of the left ilium of the subject, going around the outside of the right leg strap and along the front side of the body of the subject; from the outer side of the left ilium to the outside of the right ilium of the subject, going along the back side of the body of the subject; and from the outer side of the right ilium to the back of the left leg strap, going along the front side of the body of the subject and around the outside of the left leg strap, or vice versa. That is, the securing means may include the waist/hip strap in addition to the right leg strap and the left leg strap described above.

The securing points may be provided near both ends of the waist/hip strap.

The waist/hip strap described above exerts, on both of the right and left leg straps, a force making the right and left leg straps rotate in a direction of internal rotation. Consequently, the right and left leg straps are less likely to rotate in a direction of external rotation of the muscles of the respective right and left thighs even when the subject performs exercise such as walking with the straps worn on his/her legs. Thus, the effect of internally rotating the thigh muscles of the right leg or the left leg attained by the right and left leg straps is more likely to be maintained.

When the securing means includes the waist/hip strap described above, the upper body assisting device may further include a vertical connection member. The vertical connection member is configured so as to connect a substantial center of the portion of the upper body strap running from the left underarm to the right underarm of the subject along the back side of the body of the subject and a substantial center of the portion of the waist/hip strap running from the outside of the left ilium to the outside of the right ilium of the subject along the back side of the body of the subject. With such vertical connection member, a substantial center portion of the upper body strap running from the left underarm to the right underarm along the back side of the body of the subject is pulled downward, which further extends the spine of the subject, further correcting his/her posture. In this case, the vertical connection member may be a strip-shaped substance. In this case, the vertical connection member may be stretchable lengthwise.

Furthermore, the present inventor also proposes the following upper body assisting method as an aspect of the present invention to achieve the object of the present application.

The upper body assisting method includes the steps of: connecting a first point and a second point by an upper body strap being a strip-shaped substance having at least a length that allows the upper body strap to connect the first point, which is a predetermined point with respect to a predetermined point on a line that extends from the right greater trochanter of a subject in the forward and backward directions by 10 cm, and the second point, which is a predetermined point with respect to a predetermined point on a line that extends from the left greater trochanter of the subject in the forward and backward directions by 10 cm, with each other along a path: from the first point to the left shoulder of the subject, going along the back side of the body of the subject; from the left shoulder to the left underarm of the subject, going along the front side of the body of the subject; from the left underarm to the right underarm of the subject, going along the back side of the body of the subject; from the right underarm to the right shoulder of the subject, going along the front side of the body of the subject; and from the right shoulder to the second point, going along the back side of the body of the subject, or vice versa, the connecting the first point and the second point being achieved along the path or vice versa; and removably securing, under a state in which the upper body strap is tensioned, both ends of the upper body strap to securing points, which are located at the same positions as those of the first point and the second point or at predetermined positions where are lower than the first point and the second point and along the body of the subject.

### Brief description of the drawings

Fig. 1 is a view of a left leg strap included in an upper body assisting device according to a first embodiment (a) viewed from the front side, (b) viewed from the reverse side, and (c) viewed sideways;
Fig. 2 is a view of a waist/hip strap included in the upper body assisting device according to the first embodiment (a) viewed from the front side, (b) viewed from the reverse side, and (c) viewed sideways;
Fig. 3 is a view of an upper body strap included in the upper body assisting device according to the first embodiment (a) viewed from the front side, and (b) viewed from the reverse side;
Fig. 4 is a perspective view for use in describing how a right leg strap included in the upper body assisting device according to the first embodiment is put on the right thigh of a subject;
Fig. 5 is a perspective view right and left leg straps included in the upper body assisting device according to the first embodiment in a state where they are worn around the right and left thighs of a subject;
Fig. 6 is a view of a subject seen from the front, for use in describing how to secure both ends of the waist/hip strap to the right and left leg straps worn around the right and left thighs of the subject, respectively;
Fig. 7 is a front view of the waist/hip strap in the state where the ends thereof are secured to the right and left leg straps worn around the respective thighs of the subject;
Fig. 8 is a side view of the waist/hip strap in the state where the ends thereof are secured to the right and left leg straps worn around the respective thighs of the subject;
Fig. 9 is a back view of the waist/hip strap in the state where the ends thereof are secured to the right and left leg straps worn around the respective thighs of the subject;
Fig. 10 is a view of a subject seen from the front, for use in describing how to secure the upper body strap to the waist/hip strap;
Fig. 11 is a view of a subject seen from the back, illustrating a process of securing the upper body strap to the waist/hip strap;
Fig. 12 is a front view of a subject wearing all of the right leg strap, the left leg strap, the waist/hip strap, and the upper body strap according to the first embodiment of this application;
Fig. 13 is a side view of the subject wearing all of the right leg strap, the left leg strap, the waist/hip strap, and the upper body strap according to the first embodiment of this application;
Fig. 14 is a back view of the subject wearing all of the right leg strap, the left leg strap, the waist/hip strap, and the upper body strap according to the first embodiment of this application;
Fig. 15 is a view of a left leg strap included in an upper body assisting device according to a modified version 1 (a) viewed from the front side, and (b) viewed from the reverse side;
Fig. 16 is a view of a left leg strap included in an upper body assisting device according to a modified version 2 (a) viewed from the front side, (b) viewed from the reverse side, and (c)viewed sideways;
Fig. 17 is a perspective view for use in describing how the left leg strap included in the upper body assisting device according to the modified version 2 is put on the left thigh of a subject;
Fig. 18 is a perspective view for use in describing the left leg strap included in the upper body assisting device according to the modified version 2 in a state where it is worn around the left thigh of a subject;
Fig. 19 is a view of a subject seen from the front, for use in describing how to put an upper body assisting device according to a modified version 3 on the body of a subject; and
Fig. 20 is a view of a subject seen from the front, for use in describing how to put an upper body assisting device according to a second embodiment on the body of a subject.

### Detailed Description

Hereinafter, preferable first and second embodiments of the present invention are described in detail with reference to the drawings.

It is to be noted that corresponding parts are identified by the same reference numerals throughout the embodiments and modified versions thereof and similar descriptions will not again be made, when appropriate.

### <<First embodiment>>

An upper body assisting device according to a first embodiment is mainly made up of a right leg strap, a left leg strap, a waist/hip strap, and an upper body strap. The right leg strap, the left leg strap, and the waist/hip strap correspond to securing means in this application.

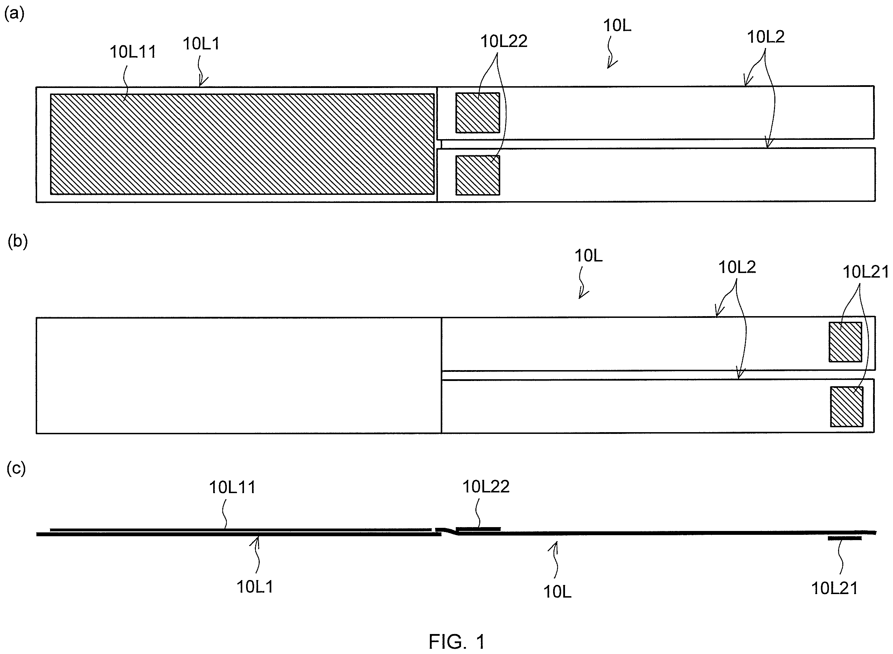

Fig. 1 shows an example of a left leg strap 10L of this application.

The left leg strap 10L is used with being wrapped around and secured to the left thigh of the subject, as described later. The left leg strap 10L compresses the thigh muscles of the subject and exerts a force on the muscles in a direction of internal rotation. The same applies to a right leg strap 10R. The right leg strap 10R is used with being wrapped around and secured to the right thigh of the subject. The right leg strap 10R compresses the thigh muscles of the subject and exerts a force on the muscles in a direction of internal rotation.

The right leg strap is in mirror symmetry to the left leg straps 10L shown in Fig. 1. Note that the left leg strap 10L shown in Fig. 1 is vertically symmetric; thus the horizontal flip of the left leg strap 10L in Fig. 1 is equivalent to the right leg strap.

Hereinafter, the construction of the left leg strap 10L is described as an example of the leg straps. The same reference numerals as those used in the following description of the left leg strap 10L, but with the suffix R instead of L, indicate parts of the right leg strap identical to those of the left one. For example, the reference numeral for the right leg strap in the following description is 10R.

The entire left leg strap 10L is formed as a strip-shaped substance. The left leg strap 10L includes, but not limited to, a rectangular main body unit 10L1 and two elongated securing units 10L2 extending to the right of the main body unit, each of which has, but not limited to, a rectangular shape in this embodiment.

The sum of the lengthwise (i.e., in the horizontal direction in Fig. 1) dimension of the main body unit 10L1 and that of the securing unit 10L2 should be longer than at least the circumference of the thigh of the subject on which the left leg strap 10L is worn (if possible, it is preferable that the total length is longer by at least 10 cm than the circumference of the thigh of the subject). In this embodiment, assuming that the length of the circumference of the thigh of the subject is approximately 35-50 cm, the sum of the lengthwise dimension of the main body unit 10L1 and that of the securing unit 10L2 is approximately 60 cm.

The main body unit 10L1 is made of cloth. The main body unit 10L1 is almost rectangular, and has a lengthwise dimension of, but not limited to, about 25 cm. This length is determined in consideration that the outer circumference of the thigh of the subject who is expected to put on the left leg strap 10L is approximately 50-60 cm. It is preferable that the length of the main body unit 10L1 is almost half the outer circumference of the thigh of the subject who is expected to put on the left leg strap 10L, more specifically, 40-50% of the outer circumference of the thigh. Further, the width (in the vertical dimension in Fig. 1) of the main body unit 10L1 is, but not limited to, about 10 cm. It is preferable that the width of the main body unit 10L1 is, but not limited to, approximately 5-15cm. With a width of ≤5 cm, the subject wearing the left leg strap 10L and the right leg strap could feel a pain. Conversely, a width of ≥15 cm prevents the subject from smoothly moving, e.g., walking.

To the front side of the main body unit 10L1, a main body securing member 10L11 is attached for achieving, in cooperation with a securing unit securing member that is described later, the purpose of securing the left leg strap 10L to the left thigh in a removable manner with the left leg strap 10L wrapped around the left thigh of the subject. The term "front side" of the left leg strap 10L indicates the surface exposed outside when the left leg strap 10L is secured to the body of the subject. The opposite is the reverse side. The definitions of the front and reverse sides also apply to other components such as the right leg strap, the waist/hip strap, and the upper body strap.

The main body securing member 10L11 is a hook-and-loop fastener and more particularly a Velcro-brand tape in this embodiment, but not limited thereto. However, the main body securing member 10L11 and the securing unit securing member may be any combinations as long as they can removably put the left leg strap 10L on the left thigh of the subject in cooperation with each other and may be replaced with, for example, a button and a button hole, two prong snap buttons, ring snap fasteners, jeans buttons and other metal interlocking discs. In this embodiment, the main body securing member 10L11 which is the hook-and-loop fastener as described above is secured to the main body unit 10L1 with a known or widely known suitable method such as adhesive or sewing.

The main body unit 10L1 is less easily stretchable lengthwise (i.e., in the horizontal direction in Fig. 1). As used herein, "less easily stretchable " indicates that, when the rate of elongation of a material similar to the main body unit 10L1 is measured using the aforementioned Measurement Method A, the rate of elongation is ≤5%. To achieve this, for example, a piece of cloth that is less easily stretchable can be used for the main body unit 10L1; or alternatively, a hook-and-loop fastener that is less easily stretchable is used and the almost entire surface of the main body unit 10L1 can be covered with the main body securing member 10L11 in the form of the hook-and-loop fastener. Velcro-brand tapes, commercially available hook-and-loop fasteners, are typically less easily stretchable in all directions. By using this as the main body securing member 10L11 and securing it to the main body unit 10L1 in such a manner that it covers the almost entire surface of the main body unit 10L1, the entire main body unit 10L1 can be made less easily stretchable lengthwise.

Each of the securing units 10L2 has an elongated rectangular shape as described above and is made of cloth. The two securing units 10L2 in the left leg strap 10L shown in Fig. 1 are exactly the same in their construction. The securing units 10L2 may be made as a single piece having the same width as the main body unit 10L1. The securing units 10L2 are secured to the main body unit 10L1 with a known or widely known suitable method such as sewing.

The width of each securing unit 10L2 is, but not limited to, approximately half the width of the main body unit 10L1. The lengthwise dimension of each securing unit 10L2 in this embodiment is, but not limited to, longer than the lengthwise dimension of the main body unit 10L1. More particularly, this length is about 35 cm.

On the reverse sides of the cloth composing the securing units 10L2, at their distal ends, securing unit securing members 10L21 are attached to the respective securing units. Each securing unit securing member 10L21 in this embodiment is a hook-and-loop fastener, and is capable of being attached to and detached from the main body securing member 10L11. The securing unit securing member 10L21 is secured to the securing unit 10L2 with a known or widely known suitable method such as sewing.

On the front sides of the securing units 10L2, in the vicinity of the proximal ends thereof, hook-and-loop fasteners, more specifically, strap front securing members 10L22 each made of a Velcro tape are attached to the respective securing units. Each strap front securing member 10L22 is, as described later, for allowing it to be secured in a removable manner to the waist/hip strap, more specifically, a waist/hip strap reverse left securing member that is also in the form of a hook-and-loop fastener provided on the waist/hip strap. In that sense, the strap front securing members 10L22 and the waist/hip strap reverse left securing members are not necessarily hook-and-loop fasteners. As in the case of the main body securing member 10L11 and the securing unit securing members 10L21, they may be replaced with, for example, a button and a button hole or metal interlocking discs. It is noted that, in the following description, a hook-and-loop fastener indicated as a way to achieve removable attachment can also be replaced with, for example, a button and a button hole or metal interlocking discs.

The securing units 10L2 are made of stretchable material that can be stretched lengthwise. With this, the left leg strap 10L is stretchable lengthwise. This helps in achieving better fitting of the left leg strap 10L onto the circumference of the thigh when the left leg strap 10L is secured to the left thigh of the subject. The stretchability of each securing unit 10L2 can be, for example, about 10-40%, preferably about 20% ± 5% in the rate of elongation when measured using the aforementioned Measurement Method A. The securing unit securing members 10L21 and the strap front securing members 10L22, which are hook-and-loop fasteners in this embodiment, provided on the securing units 10L2 occupy only a very small area of the respective securing members 10L2 at their lengthwise distal ends, as shown in Fig. 1(c). Accordingly, they do not affect the stretchability of the entire securing unit 10L2.

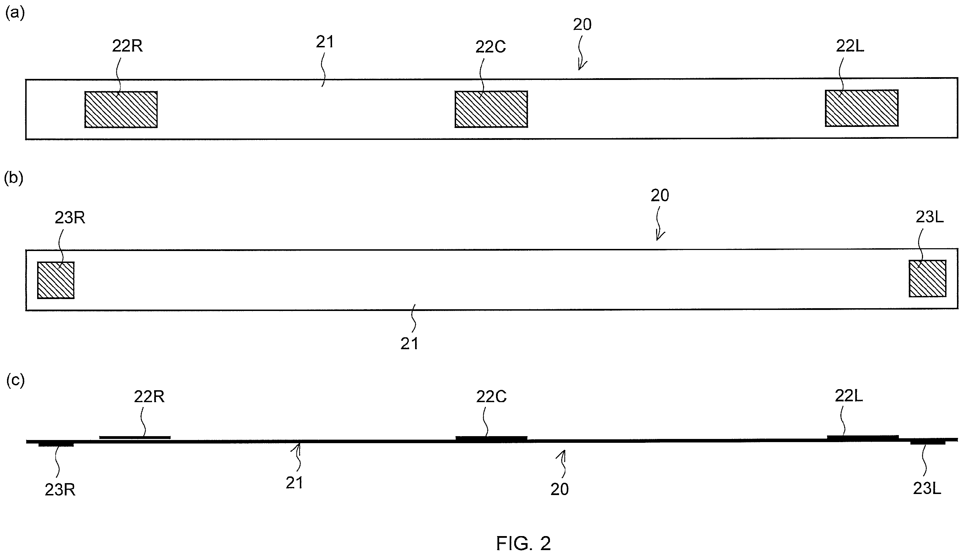

Next, the construction of the waist/hip strap is described with reference to Fig. 2.

A waist/hip strap 20 is linked to the right and left leg straps 10R and 10L worn around the respective thighs of the subject in a manner described later. The waist/hip strap 20 exerts, on the right leg strap 10R connected thereto, a force making the right leg strap 10R rotate in a direction of internal rotation, and exerts, on the left leg strap 10L connected thereto, a force making the left leg strap 10L rotate in a direction of internal rotation.

The waist/hip strap 20 in this embodiment is, but not limited to, a strip-shaped substance. The waist/hip strap 20 has a waist/hip strap main body 21. The waist/hip strap main body 21 in this embodiment has, but not limited to, an elongated rectangular shape with the same width along the entire length thereof, as shown in Fig. 2. It is preferable that the length of the waist/hip strap main body 21 is almost equal to one and a half times the waist circumference of the subject. The length in this embodiment is, but not limited to, about 150 cm. It is preferable that the width thereof is about 5-15 cm. The reason for this is the same as the reason the width of the left leg strap 10L should be 5-15 cm. The width of the waist/hip strap main body 21 in this embodiment is, but not limited to, about 5 cm. The waist/hip strap main body 21 is made of cloth that is stretchable lengthwise. The stretchability of the waist/hip strap main body 21 can be about 20-40%, preferably about 30% ± 5% in the rate of elongation when measured using the aforementioned Measurement Method A.

On the front side of the waist/hip strap main body 21 as a strip-shaped substance, at positions near the ends thereof, a waist/hip strap front right securing member 22R and a waist/hip strap front left securing member 22L both of which are hook-and-loop fasteners are secured thereto for the removable attachment to the upper body strap described later, more specifically, an upper body strap right securing member and an upper body strap left securing member of the upper body strap both of which are hook-and-loop fasteners. Their attachment to the waist/hip strap main body 21 can be made using a known or widely known technique described above. Further, on the front side of the waist/hip strap main body 21 as a strip-shaped substance, at a position near the center along the length thereof, a waist/hip strap front center securing member 22C in the form of a hook-and-loop fastener is secured to the waist/hip strap main body 21 for the removable attachment to the upper body strap described later, more specifically, an upper body strap center securing member of the upper body strap in the form of a hook-and-loop fastener. The attachment of the waist/hip strap front center securing member 22C to the waist/hip strap main body 21 can be made using a known or widely known technique described above.

On the reverse side of portions near both ends of the waist/hip strap main body 21, at positions near the ends thereof, a waist/hip strap reverse right securing member 23R for the removable attachment to a strap front securing member 10R22 of the right leg strap 10R and a waist/hip strap reverse left securing member 23L for the removable attachment to the strap front securing member 10L22 of the left leg strap 10L are provided. They are all hook-and-loop fasteners and are secured to the waist/hip strap main body 21 using a known or widely known technique described above.

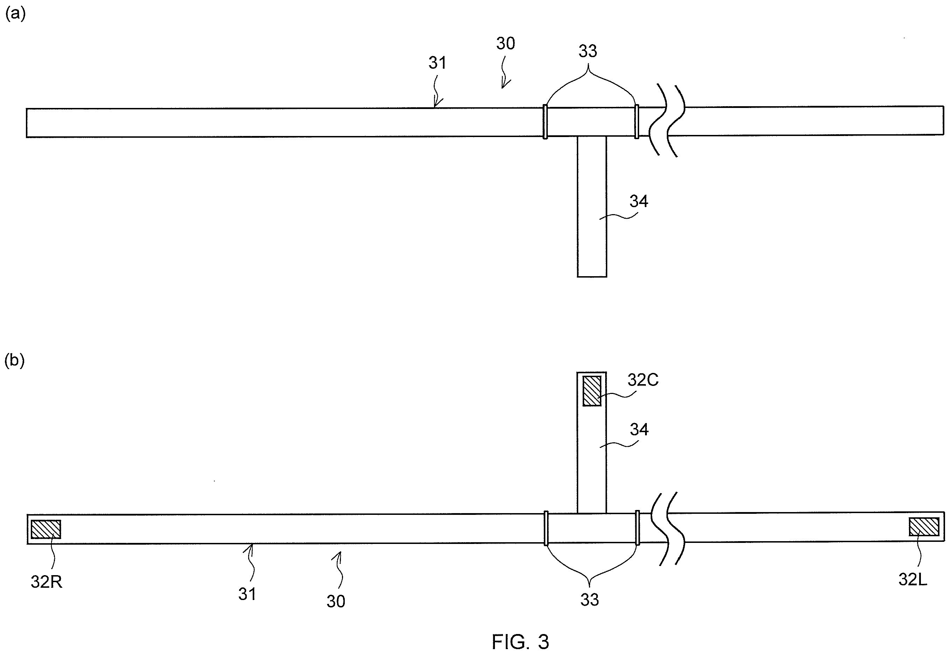

Next, the upper body strap is described with reference to Fig. 3.

The upper body strap 30 is for allowing the subject to assume an upright posture with the chest puffed out and preventing swing oscillation of the subject's body. The upper body strap 30 is connected to the waist/hip strap 20 worn around the waist of the subject in a manner described later which is connected to the right leg strap 10R and the left leg strap 10L worn around the respective thighs of the subject in a manner described later.

The upper body strap 30 in this embodiment is a strip-shaped substance. The upper body strap 30 has an upper body strap main body 31. The upper body strap main body 31 has, as shown in Fig. 3, a considerably long rectangular shape with the same width along the entire length thereof. It is preferable that the length of the upper body strap main body 31 is such that the length is sufficient for the use of the upper body strap described later. Further, as will be described later, the upper body strap main body 31 can be adjusted in its length. Broadly speaking, the length of the upper body strap main body 31 is determined between 135 cm and 200 cm, though depending on the frame of the subject who puts it on. The upper body strap 30 in this embodiment can be, but not limited to, adjusted in its length within this range. It is preferable that the width thereof is about 5-15 cm. The reason for this is the same as the reason the width of the left leg strap 10L should be 5-15 cm. The width of the upper body strap main body 31 in this embodiment is, but not limited to, about 5 cm. The upper body strap main body 31 is made of cloth that is stretchable lengthwise. The stretchability of the upper body strap main body 31 can be about 30-50%, preferably about 40% ± 5% in the rate of elongation when measured using the aforementioned Measurement Method A.

On the reverse side of the upper body strap main body 31 which is a strip-shaped substance, at positions near the ends thereof, an upper body strap right securing member 32R and an upper body strap left securing member 32L both of which are hook-and-loop fasteners are attached to the upper body strap main body 31 for removable attachment to the waist/hip strap front right securing member 22R and the waist/hip strap front left securing member 22L both of which are hook-and-loop fastener. How to secure them to the upper body strap main body 31 is according to a known or widely known technique described above.

At a position near the center of the length of the upper body strap main body 31, length adjustment members 33 are provided for adjusting the length of the upper body strap main body 31. The length adjustment members 33 can be achieved using a known or widely known technique, which are, but not limited to, two buckles in this embodiment. By adjusting the distance between these buckles, the length of the upper body strap main body 31 running between them in both directions can be adjusted, and in turn, the total length of the upper body strap main body 31 can be adjusted.

At a portion of the upper body strap main body 31 between the two buckles that constitute the length adjustment members 33, a connection band 34 is provided which extends downward in use of the upper body strap 30. The connection band 33 is a strip-shaped substance and has stretchability. Its width and stretchability are according to the upper body strap main body 31. The connection band 34 extends downward from the center between the buckles even when the distance between the buckles constituting the length adjustment members 34. On the reverse side of the connection band 34 at its lower end, an upper body strap center securing member 32C which is a hook-and-loop fastener is provided for the removable attachment to the waist/hip strap front center securing member 22C which is the hook-and-loop fastener on the waist/hip strap 20. The attachment of the upper body strap center securing member 32C to the upper body strap main body 31 is also according to a known or widely known technique described above.

Next, a method of using the upper body assisting device including the right leg strap 10R, the left leg strap 10L, the waist/hip strap 20, and the upper body strap 30 as described above and effects thereof are described.

First, the right leg strap 10R and the left leg strap 10L are secured to the respective thighs of the subject.

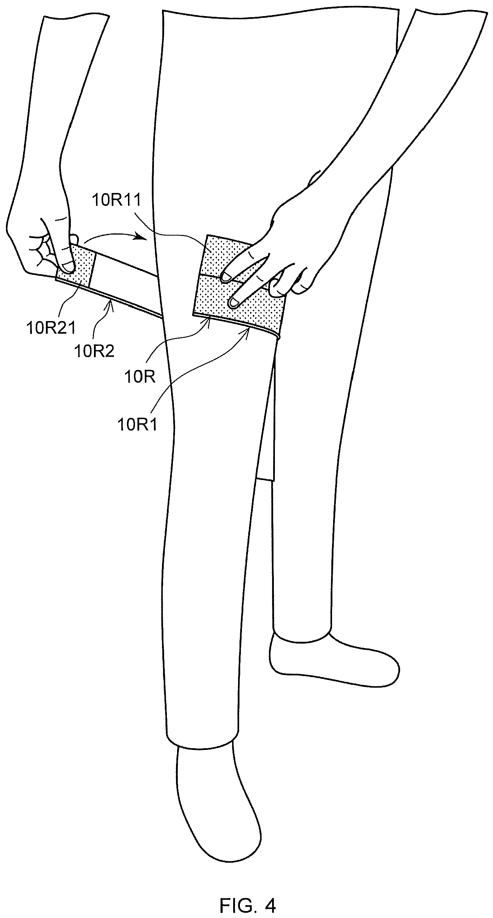

A process of securing the right leg strap 10R to the right thigh of the subject, more specifically, to the thigh at or near the top thereof is shown in Fig. 4. To secure the right leg strap 10R to the right thigh of the subject, the subject (in this embodiment, the description is made assuming that the subject puts on the upper body assisting device by himself/herself but it is of course possible that an assistant or an instructor puts the upper body assisting device on the subject; the same applies to the below) places a lengthwise end of a main body unit 10R1 of the right leg strap 10R on the front of the right thigh, presses the main body unit 10R1 gently against the right thigh with one hand to temporarily fix the position, and takes the lengthwise end of a securing unit 10R2 to the back of the right leg through between both legs, and to the front of the right thigh around the right leg. At this time, the subject applies tension to the securing unit 10R2 such that it is stretched to some extent. Then, the subject removably secure a securing unit securing member 10R21 located at the end of the reverse side of the securing unit 10R2 to a main body securing member 10R11 located in a particular range of the main body unit 10R1 on the front side at the end thereof. By securing two securing units 10R2 in this way, the right leg strap 10R is wrapped around and secured to the right thigh of the subject.

Since the entire right leg strap 10R is appropriately tensioned when the right leg strap 10R is wrapped around the right thigh, the right leg strap 10R worn around the right thigh exerts, on the right thigh muscles, a force making the right thigh muscles rotate internally.

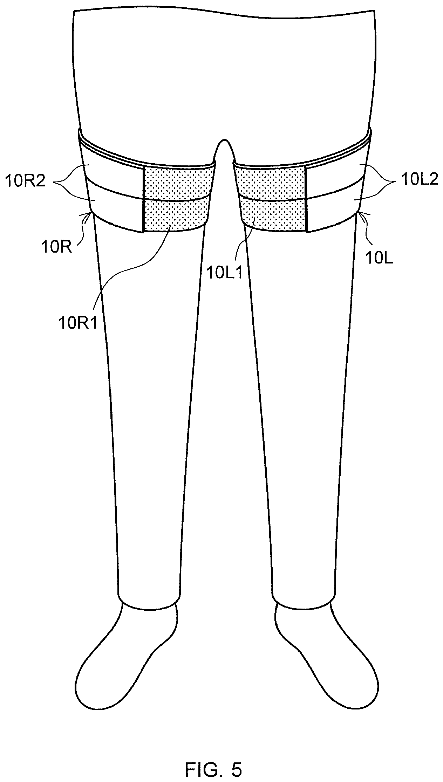

Likewise, the left leg strap 10L is secured to the left thigh of the subject at or near the top thereof. Then, the left leg strap 10L exerts, on the left thigh muscles, a force making the left thigh muscles rotate internally. Fig. 5 shows a state where the right leg strap 10R and the left leg strap 10L are worn around the respective thighs of the subject. The main body unit 10R1 of the right leg strap 10R and the main body unit 10L1 of the left leg strap 10L are both located on the back of the respective thighs.

Although it is omitted in Fig. 5, in this state, the strap front securing member 10R22 and the strap front securing members 10L22 are exposed on the back of the respective thighs.

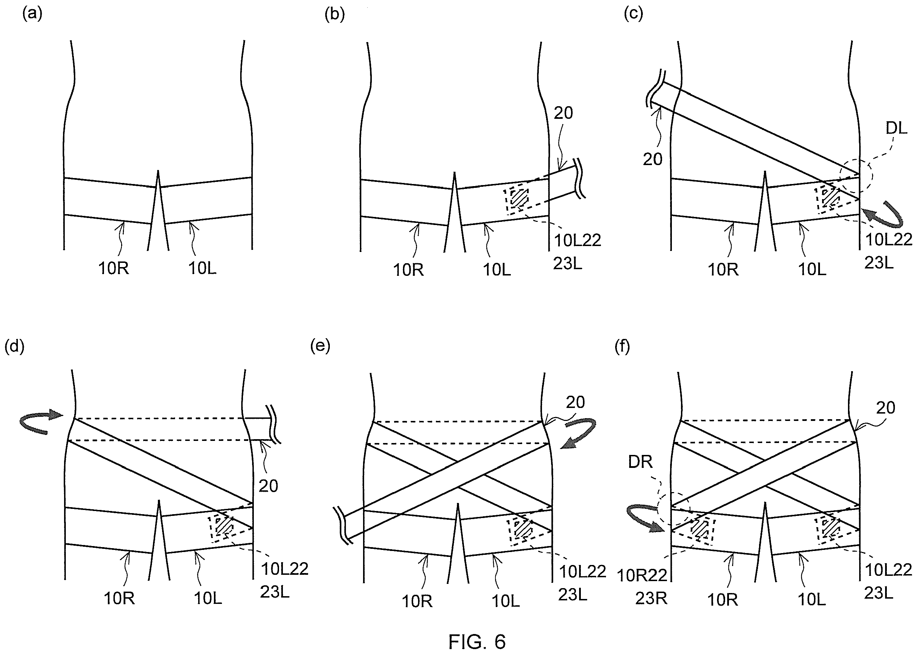

Next, the waist/hip strap 20 is secured to the right leg strap 10R and the left leg strap 10L. A way of this is shown in Fig. 6. It is noted that Fig. 6 is a view of a subject seen from the front and details of the right leg strap 10R, the left leg strap 10L, and the waist/hip strap 20 are omitted in this figure. However, the strap front securing member 10R22 and the waist/hip strap reverse right securing member 23R, and the strap front securing members 10L22 and the waist/hip strap reverse left securing member 23L, when removably secured to each other, are illustrated.

In securing the waist/hip strap 20 to the right leg strap 10R and the left leg strap 10L, in this embodiment, the subject secures the right leg strap 10R and the left leg strap 10L to the respective thighs, as shown in Fig. 6 (a) .

Next, as shown in (b) in the same figure, the subject removably secures the waist/hip strap reverse left securing member 23L of the waist/hip strap 20 to the strap front securing members 10L22 of the left leg strap 10L. In other words, the proximal end of the waist/hip strap 20 is secured to the back of the left thigh.

Next, as shown in (c) in the same figure, the subject folds the flap of the waist/hip strap 20 along the left side of the hip toward the front and brings up the flap to a front upper portion of the right ilium. Care should be taken to ensure that the waist/hip strap 20 passes near (or just over) the greater trochanter DL on the left side of the waist. The greater trochanter is a projection on the proximal end of the femur.

Next, as shown in (d) in the same figure, the subject folds the flap of the waist/hip strap 20 back in toward the ilium on the right side of the waist almost horizontally, going around the left side of the waist.

Next, as shown in (e) in the same figure, the subject folds the flap of the waist/hip strap 20 toward the front along the left side of the waist and takes it to a position near the right leg strap 10R on the front side of the body.

Next, as shown in (f) in the same figure, the subject folds the flap of the waist/hip strap 20 back in behind the right thigh and removably secures the waist/hip strap reverse right securing member 23R of the waist/hip strap 20 to the strap front securing member 10R22 of the right leg strap 10R. Care should be taken to ensure that the waist/hip strap 20 passes near (or just over) the greater trochanter DR on the right side of the waist.

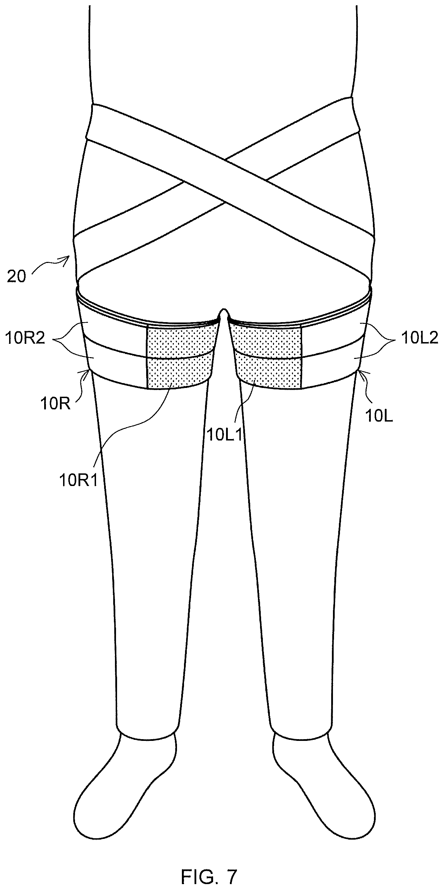

In this way, the waist/hip strap 20 is secured to the right leg strap 10R and the left leg strap 10L. Figs. 7, 8, and 9 show front, side, and back views, respectively, of the subject with the waist/hip strap 20 secured to the right leg strap 10R and the left leg strap 10L. In this state, the waist/hip strap 20 is appropriately tensioned. Consequently, a force in the counter-clockwise direction when seen from the above is exerted on the right leg strap 10R from the waist/hip strap 20, and a force in the clockwise direction when seen from the above is exerted on the left leg strap 10L from the waist/hip strap 20. These forces are for internally rotate both right and left leg straps 10R and 10L.

In securing the waist/hip strap 20 to the right leg strap 10R and the left leg strap 10L, the waist/hip strap 20 can be guided along a path in the direction reverse to the one mentioned above.

In this state, the right thigh muscles is internally rotated by the right leg strap 10R and the left thigh muscles are internally rotated by the left leg strap 10L. These internal rotations can alleviate the lower back pain or the issues with bowed legs of the subject. Furthermore, it is easier for the subject to move the legs forward during walking, more specifically, when moving the legs forward in a step-by-step movement. In contrast, each of the right leg strap 10R and the left leg strap 10L exerts a force on the thigh muscles against the motion when the leg moves backward in the step-by-step movement or when the retracted leg kicks the ground in a backward direction. Consequently, although the subject can walk naturally, he/she unintentionally performs thigh muscle strength training merely by walking because of the resistance thereof. Furthermore, it is easier for the sitting subject wearing the right leg strap 10R and the left leg strap 10L to stand up.

Besides, as described above, the right leg strap 10R and the left leg strap 10L always receive, from the waist/hip strap 20, the force making them rotate internally. Accordingly, even when the subject wearing them walks or stands up, the right thigh muscles internally rotated by the right leg strap 10R can sustain that internally rotated state well and the same applies to the left thigh muscles internally rotated by the left leg strap 10L.

Since the waist/hip strap 20 presses the greater trochanters of the subject toward the midline of the body, the body of the subject stabilizes while walking.

Next, the upper body strap 30 is connected to the waist/hip strap 20, in addition to the right leg strap 10R, the left leg strap 10L, and the waist/hip strap 20.

The upper body strap 30 is indirectly secured to the outsides of the right leg strap 10R and the left leg strap 10L when it is removably secured to the waist/hip strap 20. The upper body strap 30 can be directly secured to the outsides of the right leg strap 10R, the left leg strap 10L by providing an appropriate securing member on the outsides of the right leg strap 10R and the left leg strap 10L. In this case, there is an option to use only the right leg strap 10R, the left leg strap 10L, and the upper body strap 30 and not to use the waist/hip strap 20.

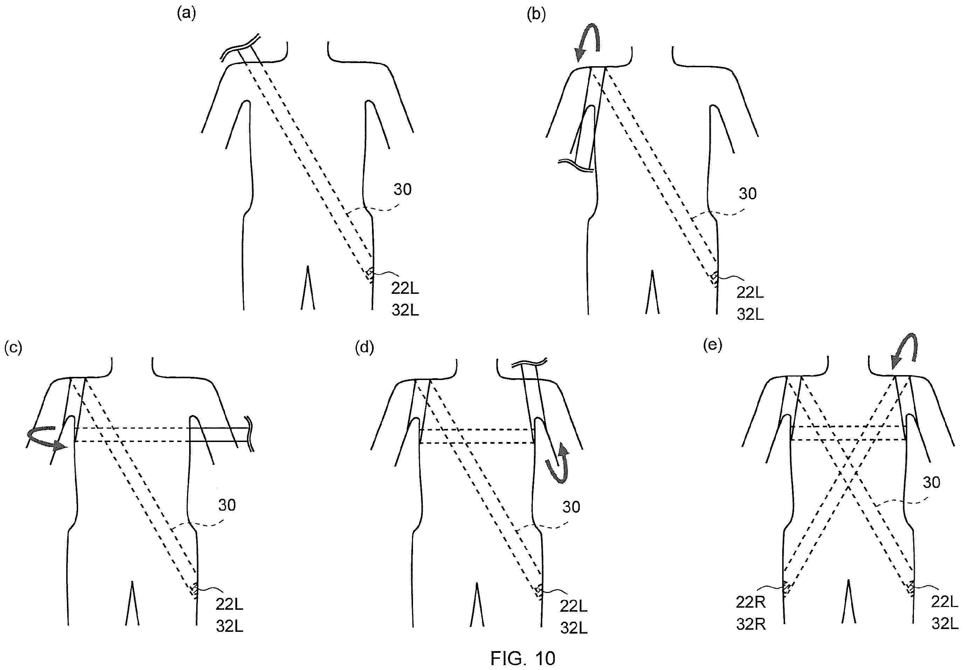

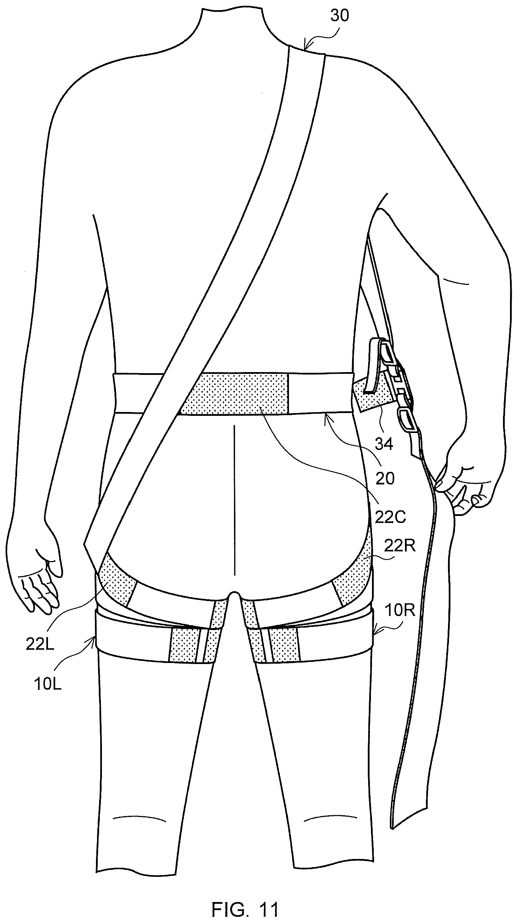

A process of securing the upper body strap 30 to the waist/hip strap 20 is shown in Figs. 10 and 11. Fig. 10 is a view of a subject seen from the front and details of the right leg strap 10R, the left leg strap 10L, and the waist/hip strap 20 are omitted in this figure for the purpose of simplifying it. However, the waist/hip strap front right securing member 22R and the upper body strap right securing member 32R, and the waist/hip strap front left securing member 22L and the upper body strap left securing member 32L, which are removably secured to each other, are illustrated.

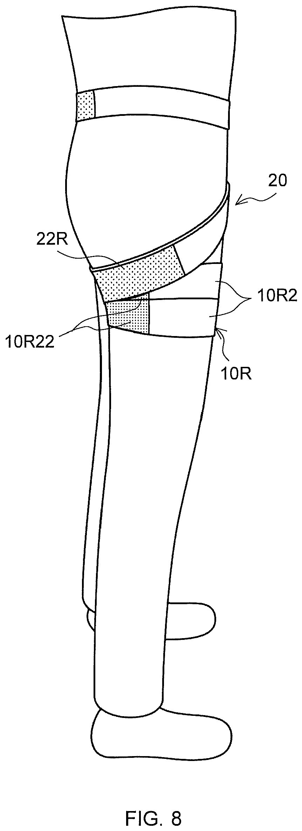

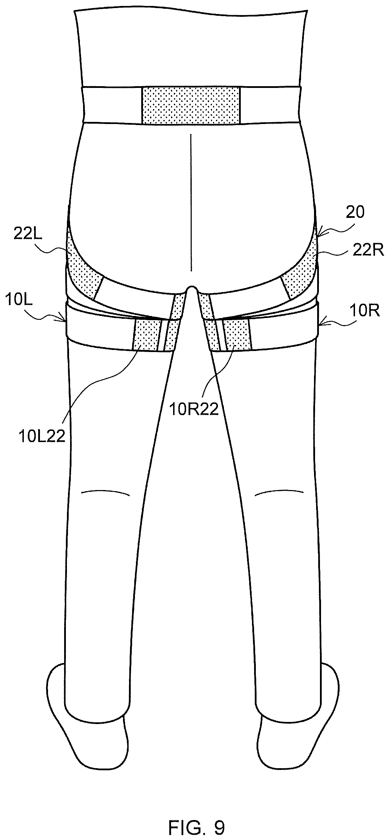

It is apparent from, for example, Figs. 8 and 9 that the waist/hip strap front right securing member 22R and the waist/hip strap front left securing member 22L are positioned on the outsides of the respective thighs.

In securing the upper body strap 30 to the waist/hip strap 20, in this embodiment, the subject first puts on the right leg strap 10R, the left leg strap 10L, and the waist/hip strap 20 as described above.

Next, as shown in Fig. 10(a), the subject removably secure the upper body strap left securing member 32L of the upper body strap 30 to the waist/hip strap front left securing member 22L of the waist/hip strap 20. Specifically, the proximal end of the upper body strap 30 is secured to the outside of the left thigh. In this embodiment, wherever the proximal end of the upper body strap 30 is secured, it comes to a position near (or just over) the left greater trochanter. The same applies to the below, and the position at which the end of the upper body strap 30 is secured serves as a securing point in the present invention. The subject takes the flap of the upper body strap 30 over the right shoulder along the back side of the body of the subject.

Next, as shown in (b) in the same figure and Fig. 11, the subject folds the flap of the upper body strap 30 along the right shoulder from the back toward the front side of the body of the subject.

Next, as shown in (c) in the same figure, the subject folds the flap of the upper body strap 30 back in toward the left underarm along the right underarm of the subject, pulling the slap almost horizontally.

Next, as shown in (d) in the same figure, the subject pulls the flap of the upper body strap 30 along the left underarm to the front side of the body of the subject, and brings up the flap in front to the left shoulder of the subject.

Next, as shown in (e) in the same figure, the subject folds back the flap of the upper body strap 30 over the left shoulder and takes the flap of the upper body strap 30 along the back side of the body to the outside of the right leg of the subject. Then, the subject secures the upper body strap right securing member 32R of the upper body strap 30 to the waist/hip strap front right securing member 22R of the waist/hip strap 20. Specifically, the distal end of the upper body strap 30 is secured to the outside of the right thigh. In this embodiment, wherever the distal end of the upper body strap 30 is secured, it comes to a position near (or just over) the right greater trochanter.

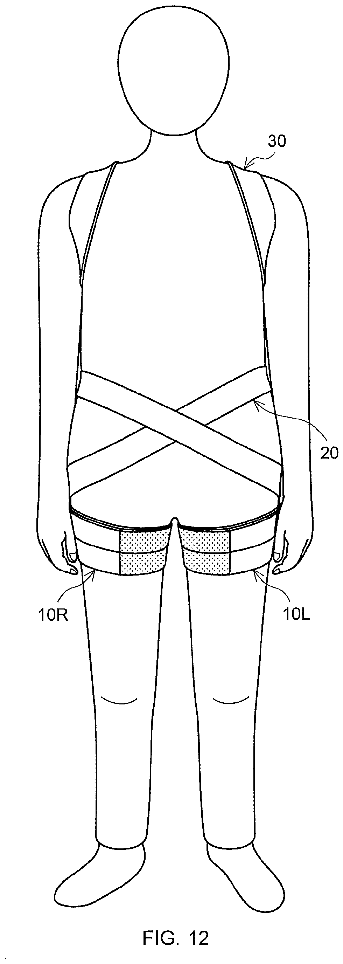

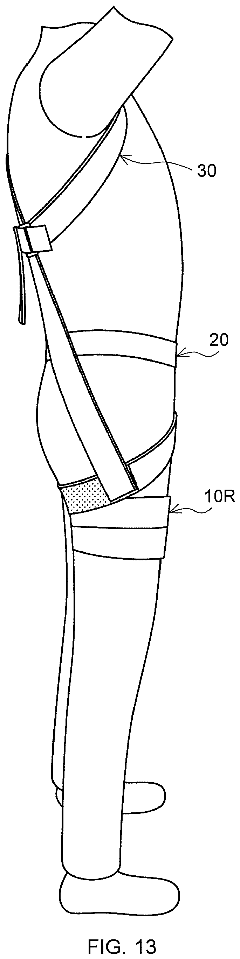

In this way, the upper body strap 30 is secured to the waist/hip strap 20 or to the right leg strap 10R and the left leg strap 10L via the waist/hip strap 20. Figs. 12, 13, and 14 show front, side, and back views, respectively, of the subject wearing the upper body strap 30 secured to the waist/hip strap 20 or to the right leg strap 10R and the left leg strap 10L via the waist/hip strap 20.

In this state, the upper body strap 30 is appropriately tensioned. The upper body strap 30 helps retract both shoulders of the subject backward and thereby allow him/her to naturally assume an upright posture with the chest puffed out. In addition, the upper body strap 30 can prevent swing and rotation of the body of the subject while walking, and thus the subject can continuously assume an upright posture, for example, even during walking. Furthermore, the upper body strap 30 assists the subject in walking.

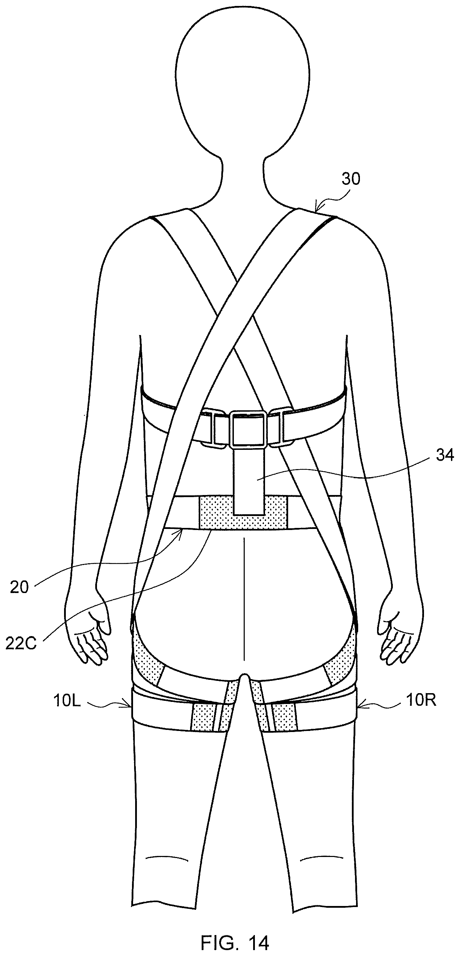

Furthermore, in securing the upper body strap 30 to the waist/hip strap 20, in particular, as shown in Fig. 14, the lower end of the connection band 34 of the upper body strap 30 which is not illustrated in Fig. 10 can be connected to the center of the almost horizontal portion of the waist/hip strap 20 that is located immediately below it. This connection is made by securing the upper body strap center securing member 32C on the reverse side of the lower end of the connection band 34 of the upper body strap 30 to the waist/hip strap front center securing member 22C. The length of the connection band 34 is slightly shorter than the distance between the almost horizontal portion of the waist/hip strap 20 and the almost horizontal portion of the upper body strap 30, both of which are in contact with the back of the subject. Therefore, when the upper body strap center securing member 32C of the upper body strap 30 is secured to the waist/hip strap front center securing member 22C of the waist/hip strap 20, the connection band 34 is tensioned. Consequently, a force making the upper body of the subject retract is exerted, which further improves the posture of the subject.

The connection band 34 in this embodiment is secured to the upper body strap 30 at the end closer to the upper body strap 30, and the end closer to the waist/hip strap 20 can be removably attached to the waist/hip strap 20. This relationship can be reversed or their ends can be adapted to be removably attached to the upper body strap 30 and the waist/hip strap 20.

The subject can stay for, for example, 20 minutes or more with the upper body assisting device including the right leg strap 10R, the left leg strap 10L, the waist/hip strap 20, and the upper body strap 30 worn, or perform light exercise such as walking. When the right leg strap 10R, the left leg strap 10L, the waist/hip strap 20, and the upper body strap 30 are not so much tensioned, the subject can stay for, for example, 2-3 hours with the straps worn while doing daily works, though there are individual differences.

### <Modified version 1>

An upper body assisting device according to a modified version 1 is described.

The upper body assisting device according to the modified version 1 is almost identical in all of its construction and how it is used to those in the first embodiment, and how it is used is completely the same.

The only difference lies in the construction of the right leg strap 10R and the left leg strap 10L included in the upper body assisting device.

As in the case of the first embodiment, the construction of the left leg strap 10 according to the modified version 1 is described for the left leg strap 10L as an example. In the modified version 1, the right leg strap 10R is in mirror symmetry to the left leg strap 10L.

The left leg strap 10L included in the upper body assisting device according to the modified version 1 has the main body unit 10L1 and the securing units 10L2 which are curved upwardly in use. Specifically, the left leg strap 10L included in the upper body assisting device according to the modified version 1 has upper and lower edges which are curved (e.g., a part of an arc or a part of an ellipse or other quadratic curve) upwardly in use. A human thigh typically gets thicker as it gets closer to the top. If the left leg strap 10L has the same width along the entire length thereof as in the case of the first embodiment, a gap may be formed between a lower portion of the left leg strap 10L and the thigh when the left leg strap 10L is wrapped around the left thigh. Then, the left leg strap 10L can fail to achieve the internal rotation of the left thigh muscles. If the aforementioned upward curve helps the left leg strap 10L fit and follow the contour of the left thigh well when the left leg strap 10L is wrapped around the left thigh, the above inconvenience can be avoided.

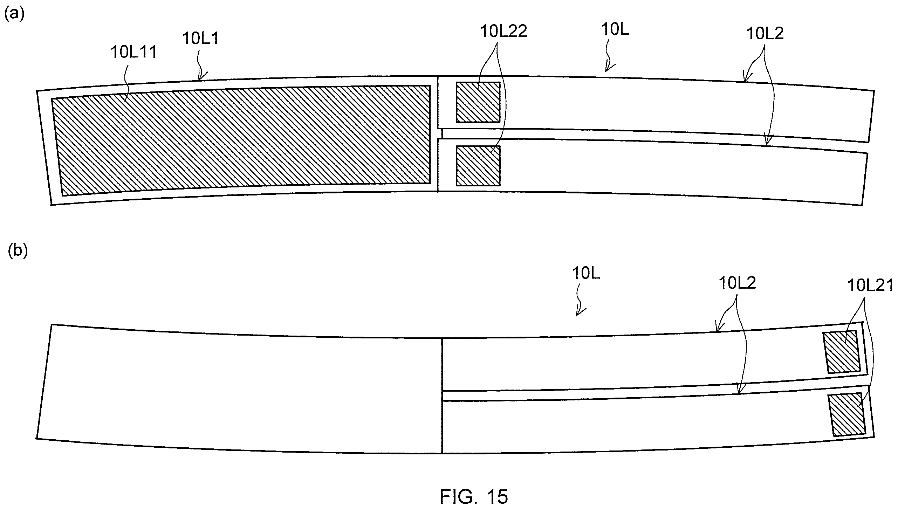

From such perspectives, it is better that the length of the upward curve along the upper edge of the left leg strap 10L is greater than the length of the upward curve along the lower edge. In this sense, it is better that the lengthwise opposite edges of the left leg strap 10L and the right leg strap are, if they are all straight, getting closer as they get closer to the bottom, as shown in Fig. 15.

### <Modified version 2>

An upper body assisting device according to a modified version 2 is described.

The upper body assisting device according to the modified version 2 is almost identical in all of its construction and how it is used to those in the first embodiment.

The only difference lies in the construction of the right leg strap 10R and the left leg strap 10L included in the upper body assisting device, and a way of putting them on the right and left thighs of the subject.

As in the case of the first embodiment, the construction of the left leg strap 10 according to the modified version 2 is described for the left leg strap 10L as an example. In the modified version 2, the right leg strap 10R is in mirror symmetry to the left leg strap 10L.

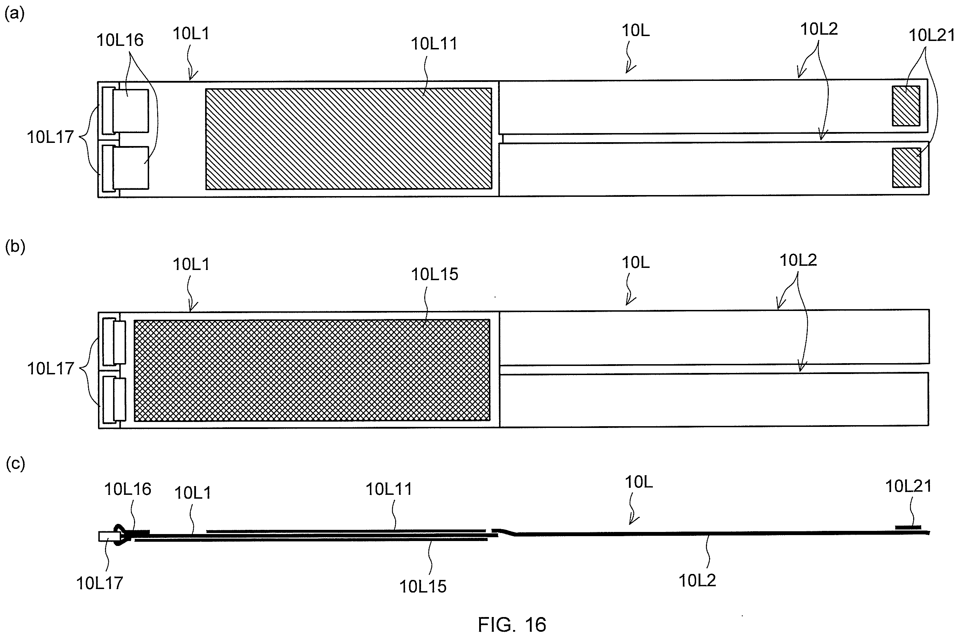

The left leg strap 10L included in the upper body assisting device according to the modified version 2 is constructed as shown in Fig. 16.

The left leg strap 10L of the modified version 2 has the main body unit 10L1 and two securing units 10L2, as in the case of the first embodiment. The width of the left leg strap 10L is similar to that in the first embodiment and the length is slightly greater than that in the first embodiment.

The length of the main body unit 10L1 in the left leg strap 10L of the modified version 2 is similar to that in the first embodiment and the length of each securing unit 10L2 is greater by about 15 cm than that in the first embodiment.

The left leg strap 10L of the modified version 2 is provided with the main body securing member 10L11 on the front side thereof. The left leg strap 10L of the modified version is provided with the securing unit securing members 10L21 that can be attached to and removed from the main body securing member 10L11, as in the case of the first embodiment. However, unlike the first embodiment, the securing unit securing members 10L21 of the modified version 2 are secured to the securing units 10L2 on the front side thereof, not the reverse side.

Furthermore, the main body unit 10L1 of the left leg strap 10L of the modified version 2 is provided with, almost over the entire reverse side, a piece of mesh sheet 10L15 which is not present in the first embodiment. The mesh sheet 10L15 is a known or widely known mesh cloth, which is used for making the left leg strap 10L breathable or making it kind to the skin when secured to the left thigh of the subject.

In addition, the left leg strap 10L of the modified version 2 does not have the strap front securing members 10L22 which are present in the first embodiment.

On one end along the length of the left leg strap 10L of the modified version 2 opposite to the end where the securing units 10L2 are attached, two buckles 10L17 are attached in such a manner that they are aligned vertically. The buckles 10L17 are formed into an elongated rectangle as a whole and have rectangular holes. The size and the shape of the holes in the two, upper and lower buckles 10L17 are such that the securing units 10L2 aligned vertically can pass through.

Although the buckles 10L17 can be secured to the main body unit 10L1 by any means, each buckle 10L17 in this embodiment is secured by stitching both ends of a piece of cloth 10L16 that is passed through the buckle 10L17 and flipped into half to the ends of the front and reverse sides of the main body unit 10L1.

The right leg strap 10R and the left leg strap 10L are different from those of the first embodiment in how it is used, particularly a way of putting them on the thigh of the subject.

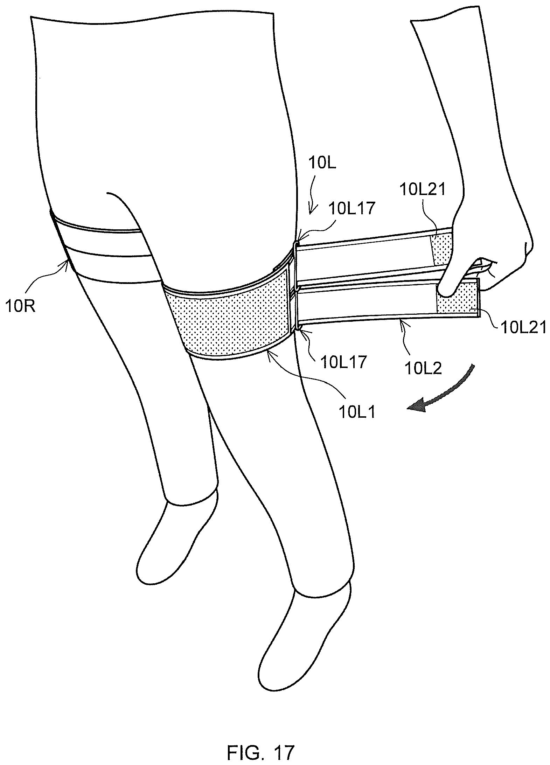

By way of example, a way of putting the left leg strap 10L on the left thigh of the subject is described. For this description, Fig. 17 is used.

In order to put the left leg strap 10L of the modified version 2 on the left thigh, the left leg strap 10L is passed through between both legs with the main body unit 10L1 placed on the back and the securing units 10L2 on the front, and the reverse side thereof facing the left thigh. In this state, while adjusting the buckles 10L17 to positions behind the outside of the left leg, the flaps of the upper and lower securing units 10L2 are passed through the respective buckles 10L17. Fig. 17 shows that state. Then, the flaps of the securing units 10L2 are pulled toward the front of the legs as indicated by the arrow in the figure, and the securing unit securing members 10L21 at the ends of the respective securing units 10L2 are removably secured to the main body securing member 10L11. In this case, when the left leg strap 10L is secured to the left thigh, a force is exerted in a direction from behind the outside of the left leg to the front inwardly (i.e., the securing units 10L2 are pulled), whereby the left thigh muscles rotate internally.

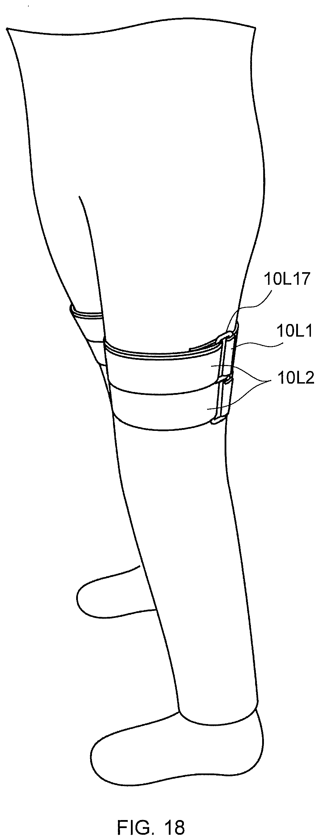

Fig. 18 shows the left leg strap 10L secured to the left thigh.

In summary, unlike the first embodiment, in the left leg strap 10L of the modified version 2, the securing unit 10L2 passed through the buckle 10L17 is flipped at the buckle 10L17 and then secured to the main body securing member 10L11. Because of this flip, unlike the first embodiment, in the left leg strap 10L of the modified version 2, the securing unit securing member 10L21 is secured to the front side of the securing unit 10L2.

Subsequent processes of the method of using the upper body assisting device according to the modified version 2 are similar to those in the first embodiment. Specifically, as in the first embodiment, the upper body assisting device according to the modified version 2 can be used in combination with the right leg strap 10R, the left leg strap 10L, the waist/hip strap 20, and the upper body strap 30.

It is noted that the left leg strap 10L of the upper body assisting device according to the modified version 2 does not have the strap front securing members 10L22 which are used to connect the waist/hip strap 20 and the left leg strap 10L. However, in the left leg strap 10L of the modified version 2, even when the securing unit securing members 10L21 of the securing units 10L2 are attached to the main body securing member 10L11 covering almost entire surface of the front side of the main body unit 10L1, a portion of the main body securing member 10L11 closer to the securing units 10L2 is exposed without being covered with the securing units 10L2. The exposed portion of the main body securing member 10L11 is located immediately behind the buckles 10L17, i.e., on the back of the left leg. By using the main body securing member 10L11 exposed immediately behind the buckles 10L17 in place of the strap front securing members 10L22 in the first embodiment, the removable attachment between the waist/hip strap 20 and the left leg strap 10L can also be achieved in the upper body assisting device according to the modified version 2.

The removable attachment between the waist/hip strap 20 and the right leg strap 10R can also be achieved in a similar manner.

The removable attachment of the upper body strap 30 to the waist/hip strap 20 is identical to that of the first embodiment.

### <Modified version 3>

An upper body assisting device according to a modified version 3 is almost identical in its construction to that of the upper body assisting device according to the first embodiment. As in the case of the first embodiment, the upper body assisting device according to the modified version 3 includes the right leg strap 10R, the left leg strap 10L, and the upper body strap 30. However, unlike the first embodiment, the upper body assisting device according to the modified version 3 does not include the waist/hip strap 20.

The right leg strap 10R and the left leg strap 10L of the upper body assisting device according to the modified version 3 may be identical to those in the first embodiment (including those in the modified versions 1 and 2). However, the right leg strap 10R and the left leg strap 10L of the modified version 3 include, on the front side portions thereof when the right leg strap 10R and the left leg strap 10L are worn around the right and left thighs of the subject, securing members (omitted in the figure) for securing the upper body strap right securing member 32R of the upper body strap 30 to the right leg strap 10R and securing the upper body strap left securing member 32L of the upper body strap 30 to the left leg strap 10L. The securing members may have a construction similar to those of the strap front securing members 10R22 and 10L22 except for positions thereof.

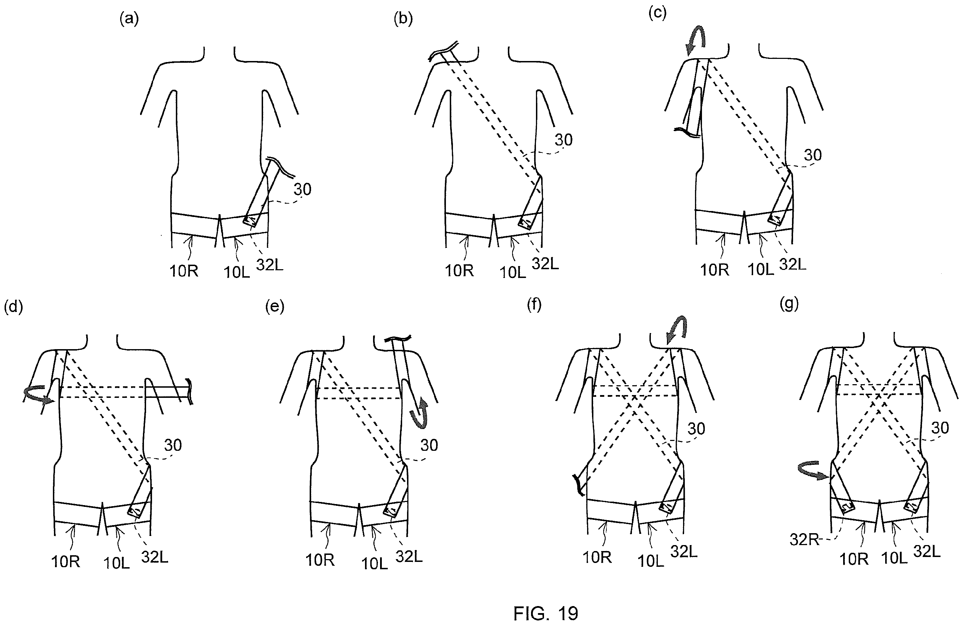

A process of securing the upper body assisting device according to the modified version 3 to the right leg strap 10R and the left leg strap 10L corresponding to the securing means of the present invention in the modified version 3 is shown in Fig. 19. Fig. 19 is a view of a subject as seen from the front and details of the above-mentioned securing members of the right leg strap 10R and the left leg strap 10L are omitted in this figure for the purpose of simplifying it. However, the upper body strap right securing member 32R and the upper body strap left securing member 32L are illustrated, which are removably secured to the above-mentioned securing members of the right leg strap 10R and the left leg strap 10L, respectively.

First, the subject wears the right leg strap 10R and the left leg strap 10L as in the case of the first embodiment.

Next, as shown in Fig. 19(a), the subject removably secures the upper body strap left securing member 32L of the upper body strap 30 to the securing member, which is omitted in the figure, of the left leg strap 10L. Specifically, the proximal end of the upper body strap 30 is secured to the front side of the right thigh.

Next, as shown in (b) in the same figure, the subject takes the flap of the upper body strap 30 to the upper side of the left waist and the outer side of the ilium along the front side of the body of the subject. Then, the subject takes the flap of the upper body strap 30 to the back of the body of the subject, and takes the flap of the upper body strap 30 over the right shoulder along the back side of the body of the subject. At this time, the upper body strap 30 comes to a position somewhat in front of the left greater trochanter, specifically, about 5 cm in front of the greater trochanter.

After that, the procedures similar to those of the first embodiment are performed for a while. Next, as shown in (c) in the same figure, the subject takes the flap of the upper body strap 30 along the right shoulder from the back side toward the front side of the body of the subject.

Next, as shown in (d) in the same figure, the subject takes the flap of the upper body strap 30 to the back side of the body of the subject along the right underarm of the subject, and brings the flap to the left underarm of the subject by pulling the flap almost horizontally.

Next, as shown in (e) in the same figure, the subject pulls the flap of the upper body strap 30 along the left underarm to the front side of the body of the subject, and brings up the flap to the left shoulder of the subject along the front side of the body of the subject.

Next, as shown in (f) in the same figure, the subject folds back the flap of the upper body strap 30 over the left shoulder to take the flap of the upper body strap 30 to the back side of the body, and takes the flap of the upper body strap 30 to the upper side of the right waist and the outer side of the ilium along the back side of the body of the subject.

Next, as shown in (g) in the same figure, the subject takes the flap of the upper body strap 30 to the front side of the right waist, and thereafter folds the flap of the upper body strap 30 to the front side of the right leg strap 10R. Then, the subject secures the upper body strap right securing member 32R to the securing member, which is omitted in the figure, of the right leg strap 10R. At this time, the upper body strap 30 comes to a position somewhat in front of the right greater trochanter, specifically, about 5 cm in front of the greater trochanter.

As described above, it is not required that the upper body strap 30 pass just over the right and left greater trochanters. When the upper body strap 30 passes just over the right and left greater trochanters, the effect of the upper body assisting device becomes greater. When the both ends of the upper body strap 30 are placed on the back side of the right leg strap 10R and the left leg strap 10L, the upper body strap 30 passes slightly behind the right and left greater trochanters. As is similarly applied to the cases of another embodiment and the modified versions, it is only required that the upper body strap 30 pass just over the greater trochanter or within a range of 10 cm in front and behind the greater trochanter.

With this, the procedure of putting the upper body strap 30 and the upper body assisting device on the subject is terminated. In this case, the upper body strap 30 does not apply the force of internal rotation to both the right leg strap 10R and the left leg strap 10L. The right leg strap 10R and the left leg strap 10L in this case are used only for securing the upper body strap 30.

### <<Second embodiment>>

An upper body assisting device according to a second embodiment is described.

The upper body assisting device according to the second embodiment is, in one word, a device in which the right leg strap 10R and the left leg strap 10L included in the upper body assisting device according to the modified version 3 of the first embodiment are united to form shorts 19.

In the upper body assisting device according to the second embodiment, the united shorts 19 correspond to the securing means in this application.

At predetermined positions on the front side of the shorts 19, for example, at positions on the right leg strap 10R and the left leg strap 10L at which the securing members are provided in the modified version 3 of the first embodiment, there are provided securing members constructed similarly to those in the modified version 3 of the first embodiment, which are omitted in the figures.

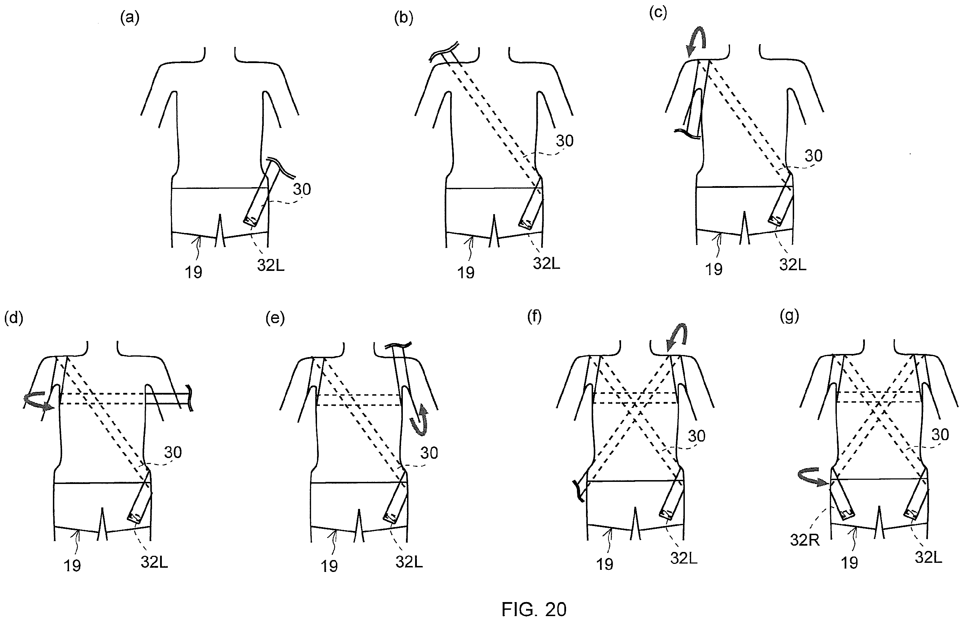

Thus, when this upper body assisting device is to be used, the subject wears the shorts 19, and both ends of the upper body strap 30 can be attached to securing members, which are omitted in the figures, by the method similar to that in the case of the modified version 3 of the first embodiment (Fig. 20).

Moreover, for example, the securing members for securing the upper body strap right securing member 32R and the upper body strap left securing member 32L of the upper body strap 30 to the shorts 19 can also be provided at positions on the surface of the shorts 19 at which the waist/hip strap front right securing member 22R and the waist/hip strap front left securing member 22L are provided in the first embodiment. In such case, although not shown in the figures, the both ends of the upper body strap 30 can be secured to the shorts 19 by the method illustrated in Fig. 10.

Further, when the surface of the shorts 19 is entirely formed as the securing member, the both ends of the upper body strap 30 can be secured to the shorts 19 by any one of the method illustrated in Fig. 10 and the method illustrated in Fig. 20. Furthermore, the both ends of the upper body strap 30 can be secured to anywhere on the surface of the shorts 19.

### Denotation of symbols

- 10R: right leg strap
- 10R1: main body unit
- 10R11: main body securing member
- 10R2: securing unit
- 10R21: securing unit securing member
- 10R22: strap front securing member
- 10L: left leg strap
- 10L1: main body unit
- 10L11: main body securing member
- 10L2: securing unit
- 10L21: securing unit securing member
- 10L22: strap front securing member
- 19: shorts
- 20: waist/hip strap
- 21: waist/hip strap main body
- 22R: waist/hip strap front right securing member
- 22L: waist/hip strap front left securing member
- 22C: waist/hip strap front center securing member
- 23R: waist/hip strap reverse right securing member
- 23L: waist/hip strap reverse left securing member
- 30: upper body strap
- 31: upper body strap main body
- 32R: upper body strap right securing member
- 32L: upper body strap left securing member
- 32C: upper body strap center securing member
- 34: connection band

## Claims

1. An upper body assisting device comprising:
an upper body strap being a strip-shaped substance having at least a length that allows the upper body strap to connect a first point, which is a predetermined point with respect to a predetermined point on a line that extends from the right greater trochanter of a subject in the forward and backward directions by 10 cm, and a second point, which is a predetermined point with respect to a predetermined point on a line that extends from the left greater trochanter of the subject in the forward and backward directions by 10 cm, with each other along a path:
from the first point to the left shoulder of the subject, going along the back side of the body of the subject;
from the left shoulder to the left underarm of the subject, going along the front side of the body of the subject;
from the left underarm to the right underarm of the subject, going along the back side of the body of the subject;
from the right underarm to the right shoulder of the subject, going along the front side of the body of the subject; and
from the right shoulder to the second point, going along the back side of the body of the subject, or vice versa; and
securing means configured to removably secure, under a state in which the upper body strap is tensioned, both ends of the upper body strap to securing points, which are located at the same positions as those of the first point and the second point or at predetermined positions where are lower than the first point and the second point and along the body of the subject.

2. The upper body assisting device according to Claim 1, wherein the upper body strap is stretchable in a lengthwise direction thereof.

3. The upper body assisting device according to Claim 1,
wherein the securing means is cloth to be secured to the body of the subject, and
wherein the securing points are provided on the cloth.

4. The upper body assisting device according to any one of Claims 1 to 3,
wherein the securing means is a strip-shaped substance, and
wherein the securing points are provided on the strip-shaped substance being secured to the body of the subject in a state of being wrapped around the body of the subject.

5. The upper body assisting device according to Claim 4,
wherein the securing means being the strip-shaped substance includes:
a right leg strap to be secured in a state of being wrapped around the right thigh; and
a left leg strap to be secured in a state of being wrapped around the left thigh, and
wherein the securing points are provided on the right leg strap and the left leg strap, respectively.

6. The upper body assisting device according to Claim 4,
wherein the securing means being the strip-shaped substance includes:
a right leg strap to be secured in a state of being wrapped around the right thigh;
a left leg strap to be secured in a state of being wrapped around the left thigh; and
a waist/hip strap being a strip-shaped substance having a length that allows the waist/hip strap to connect the back of the right leg strap worn on the right leg of the subject and the back of the left leg strap worn on the left leg of the subject with each other along a path:
from the back of the right leg strap to the outer side of the left ilium of the subject, going around the outside of the right leg strap and along the front side of the body of the subject;
from the outer side of the left ilium to the outside of the right ilium of the subject, going along the back side of the body of the subject; and
from the outer side of the right ilium to the back of the left leg strap, going along the front side of the body of the subject and around the outside of the left leg strap, or vice versa, and
wherein the securing points are provided near both ends of the waist/hip strap.

7. The upper body assisting device according to Claim 6, further comprising a vertical connection member, the vertical connection member being configured so as to connect a substantial center of the portion of the upper body strap running from the left underarm to the right underarm along the back side of the body of the subject and a substantial center of the portion of the waist/hip strap running from the outer side of the left ilium to the outer side of the right ilium along the back side of the body of the subject.

8. An upper body assisting method comprising the steps of:
connecting a first point and a second point by an upper body strap being a strip-shaped substance having at least a length that allows the upper body strap to connect the first point, which is a predetermined point with respect to a predetermined point on a line that extends from the right greater trochanter of a subject in the forward and backward directions by 10 cm, and the second point, which is a predetermined point with respect to a predetermined point on a line that extends from the left greater trochanter of the subject in the forward and backward directions by 10 cm, with each other along a path:
from the first point to the left shoulder of the subject, going along the back side of the body of the subject;
from the left shoulder to the left underarm of the subject, going along the front side of the body of the subject;
from the left underarm to the right underarm of the subject, going along the back side of the body of the subject;
from the right underarm to the right shoulder of the subject, going along the front side of the body of the subject; and
from the right shoulder to the second point, going along the back side of the body of the subject, or vice versa, the connecting the first point and the second point being achieved along the path or vice versa; and
removably securing, under a state in which the upper body strap is tensioned, both ends of the upper body strap to securing points, which are located at the same positions as those of the first point and the second point or at predetermined positions where are lower than the first point and the second point and along the body of the subject.
